# EUROPÄISCHE PATENTSCHRIFT

(11) **EP 2 285 359 B1**
(45) Veröffentlichungstag und Bekanntmachung des Hinweises auf die Patenterteilung: **05.09.2012**
(21) Anmeldenummer: 09741812.3
(22) Anmeldetag: 23.04.2009
(51) Int. Cl.: A61K 9/22, A61K 31/4152, A61K 31/495, A61K 31/662, A61K 31/7048, A61K 38/15

(54) **FESTE ARZNEIMITTELFORMULIERUNG MIT VERZÖGERTER FREISETZUNG**
SOLID PHARMACEUTICAL FORMULATION WITH DELAYED RELEASE
FORMULATION PHARMACEUTIQUE SOLIDE À LIBÉRATION RETARDÉE

(30) Priorität: 07.05.2008 DE 102008022520
(43) Veröffentlichungstag der Anmeldung: 23.02.2011
(73) Patentinhaber: Bayer Animal Health GmbH, 51368 Leverkusen (DE)
(72) Erfinder: KANIKANTI, Venkata-Rangarao, 51381 Leverkusen (DE); BACH, Thomas, 42349 Wuppertal (DE); TRÄUBEL, Michael, 50733 Köln (DE); ALTREUTHER, Gertraut, 42105 Wuppertal (DE); REHAGEN, Martina, 53797 Lohmar (DE); SCHMIDT, Axel, 42329 Wuppertal (DE)
(74) Vertreter: Storch, Matthias
(86) Internationale Anmeldenummer: PCT/EP2009/002951
(87) Internationale Veröffentlichungsnummer: WO 2009/135593

(56) Entgegenhaltungen:
- EP-A1- 0 439 858
- EP-A1- 0 545 209
- WO-A1-02/00202
- WO-A1-93/21907
- WO-A1-99/51201
- WO-A1-2004/014346
- WO-A1-2005/044271
- WO-A1-2008/013197
- WO-A2-03/005951
- DE-A1- 19 531 684
- DE-A1- 19 920 415
- DE-A1-102006 044 694
- US-A- 6 068 859
- US-A1- 2004 208 925
- US-A1- 2005 032 718
- US-A1- 2005 053 658
- HARDY ET AL: "Modulation of drug release kinetics from hydroxypropyl methyl cellulose matrix tablets using polyvinyl pyrrolidone" INTERNATIONAL JOURNAL OF PHARMACEUTICS, ELSEVIER BV, NL LNKD- DOI:10.1016/J.IJPHARM.2007.01.026, Bd. 337, Nr. 1-2, 19. Mai 2007 (2007-05-19), Seiten 246-253, XP022085275 ISSN: 0378-5173
- SHAO Z J ET AL: "EFFECTS OF FORMULATION VARIABLES AND POST-COMPRESSION CURING ON DRUG RELEASE FROM A NEW SUSTAINED-RELEASE MATRIX MATERIAL: POLYVINYLACETATE-POVIDONE" PHARMACEUTICAL DEVELOPMENT AND TECHNOLOGY, NEW YORK, NY, US LNKD- DOI:10.1081/PDT-100002201, Bd. 6, Nr. 2, 1. Januar 2001 (2001-01-01), Seiten 247-254, XP008033356 ISSN: 1083-7450
- GULDE C ET AL: "Pharmaceutical and biologic availability of chlorpromazine from macromolecule-containing tablets" DIE PHARMAZIE, GOVI VERLAG PHARMAZEUTISCHER VERLAG GMBH, ESCHBORN, DE, Bd. 38, Nr. 8, 1. August 1983 (1983-08-01) , Seiten 542-546, XP008126331 ISSN: 0031-7144
- FIONA MCINNES ET AL: "In Vivo Performance of an Oral MR Matrix Tablet Formulation in the Beagle Dog in the Fed and Fasted State: Assessment of Mechanical Weakness" PHARMACEUTICAL RESEARCH, KLUWER ACADEMIC PUBLISHERS-PLENUM PUBLISHERS, NL, Bd. 25, Nr. 5, 5. Oktober 2007 (2007-10-05), Seiten 1075-1084, XP019613050 ISSN: 1573-904X in der Anmeldung erwähnt
- Anonymous: "Pink wirkt! Catosal wirkt kolossal." Bayer Health Care Dezember 2007 (2007-12), XP002606355 Gefunden im Internet: URL:http://www.catosal.com/index.php/fusea ction/download/lrn_file/germany-catosal-fo lder.pdf [gefunden am 2010-10-21]

## Beschreibung

Die Erfindung betrifft eine feste Arzneimittelzubereitung mit verzögerter Freisetzung der Wirkstoffe, die sich insbesondere zur Anwendung bei Tieren eignet.

In der Tiermedizin und insbesondere für Katzen und Hunde sind Arzneimittel mit verzögerter Freisetzung ("controlled release"-Formulierungen, Retardformulierungen) nicht üblich, obwohl für die Anwendung beim Menschen zahlreiche auf verschiedenen Techniken basierende Retard-Arzneimittel verfügbar sind. Einer der Hauptgründe dafür ist, dass Tiere, vor allem Katzen und Hunde, sich vom Menschen hinsichtlich der Verweilzeiten im Gastrointestinaltrakt (gastrointestinal transit times [GITT]), Nahrungsmitteleinflüssen, Einfluß der Freßgewohnheiten, Art, Größe, pH-Wert im Magen, Darmenzyme, Permeabilität des Gastrointestinaltraktes und der Bereiche, in denen Wirkstoffe absorbiert werden, unterscheiden [S.C. Sutton, Adv. Drug delivery reviews, 56 (2004) 1383 - 1398]. Physiologische Unterschiede zwischen Hunden und Menschen sind in der Literatur ausführlich beschrieben [Dressman, Pharm Res., 3 (1986) 123 - 131; Schneider et al., J. Med. Chem., 42 (1999) 5072]. Wenn ein Tier eine übliche Retardtablette bei der oralen Aufnahme zerkaut oder sonstwie zerkleinert, wird der Wirkstoff sehr schnell freigesetzt und der eigentliche Zweck der Retardtablette wird nicht erreicht. Die Aufnahme des Wirkstoffs oder - anders ausgedrückt - das pharmakokinetische Profil kann erheblich durch das Zerkauen der Tablette durch das Tier verändert werden. Ziel ist es für solche Fälle eine Formulierung zu entwickeln, bei der die Zerkleinerung einen möglichst geringen Einfluss auf die Aufnahme des Wirkstoffs hat. Die Entwicklung einer solchen Formulierung für die Anwendung beim Tier stellt daher für den Fachmann ein schwieriges technisches Problem dar. Dabei ist auch zu beachten, dass die mechanische Belastung z. B. in einem Hundemagen wesentlich höher ist als beim Menschen.

In WO 2004/014346 werden Carprofen-Tabletten mit verzögerter Freisetzung beschrieben, in denen die hydrophilen Polymere Methocel (Hydroxypropylmethylcellulose "HPMC"), Polyox und Carbopol eingesetzt werden. Die Tabletten beruhen auf dem Prinzip, dass sie Mikropartikel enthalten, die selbst "controlled-release" Eigenschaften haben. Solche Formulierungen sind aufwendig in der Herstellung, außerdem bleibt unklar, ob ein geeignetes Freisetzungsprofil im Gastrointestinaltrakt des Tieres ohne Einbußen bei der Bioverfügbarkeit erreicht werden kann.

In EP 0439858 A1 werden Phenytoin-Tabletten mit verzögerter Freisetzung zur Anwendung an Tieren beschrieben, welche als Gelbildner Zellulose- oder Stärkederivate, Polyvinylpyrrolidon oder Polyvinylalkohol enthalten.

Eine weitere Schwierigkeit für den Galeniker besteht darin, dass die Verweilzeiten im Magen und im Verdauungstrakt erheblich schwanken können. Die Verweilzeiten im Gastrointestinaltrakt nüchterner und gefütterter Beagle-Hunde variieren erheblich [siehe z. B. die Figuren 7 und 8 in Sutton a.a.O.]. Sutton gibt an, dass etwa 80% der Tabletten eine Verweildauer im Verdauungstrakt von über 24 Stunden hatten. Daraus schließt er, dass mit Retardformulierungen, die eine *in vitro* Freisetzungsdauer von weniger als 24 Stunden aufweisen, üblicherweise die gesamte Dosis vom Hund aufgenommen werden kann und nicht vorzeitig ausgeschieden wird. Weiterhin hängt die Verweildauer im Magen auch von der Größe des Arzneimittels und der Art und Rasse des betreffenden Tieres ab [siehe z. B. Fix et al., Pharm. Res., 10 (1993) 1087 -1089]. Ziel ist es eine Formulierung zu entwickeln, die sich für die Anwendung bei verschiedenen Tierarten und -rassen eignet.

Eine weitere Schwierigkeit für den Galeniker ergibt sich daraus, dass der Wirkstoff oft nur in bestimmten begrenzten Bereichen des Gastrointestinaltraktes überhaupt aufgenommen werden kann. Wird der Wirkstoff beispielsweise nur im Dünndarm absorbiert, sollte die Formulierung den Wirkstoff auch möglichst vollständig im Dünndarm freisetzen. Schwankungen der Verweildauer der Formulierung im Gastrointestinaltrakt können die Bioverfügbarkeit beeinflussen. Den Verdauungstrakt z. B. bei Hunden durchläuft in bestimmten Zeitabständen eine peristaltische Bewegung, die auch als *"housekeeper wave"* bezeichnet wird; diese *housekeeper wave* hat einen Einfluss auf die Verweildauer der Formulierung im Gastrointestinaltrakt, denn die Verweildauer hängt davon ab, ob die *housekeeper wave* gerade begonnen hat oder erst später einsetzt. Verweilzeiten im Magen hängen auch erheblich von Art und Menge der aufgenommenen Nahrung und sogar von der Größe der Magenpförtneröffnung ab.

Diese Schwierigkeiten mögen dazu beigetragen haben, dass bis heute nach unserer Kenntnis keine oralen Arzneimittel mit kontrollierter Freisetzung für Hunde auf dem Markt sind. Jedenfalls ist die Entwicklung einer Formulierung mit kontrollierter Freisetzung, die sich z. B. für Katzen und/oder Hunde eignet, eine schwierige Aufgabe, deren Lösung der Literatur nicht ohne weiteres entnommen werden kann.

Zahlreiche Möglichkeiten eine verzögerte Freisetzung zu erreichen sind bekannt. Üblicherweise bevorzugt der Fachmann Matrixtabletten, die ein Polymer wie z. B. Celluloseether (Hydroxypropylcellulose oder Hydroxypropylmethylcellulose) enthalten, das ein hydrophiles Gel bildet, da solche Tabletten mit den in der pharmazeutischen Industrie üblichen Maschinen hergestellt werden können und auch unempfindlich bezüglich der Herstellungsbedingungen sind. Selbst wenn z. B. ein Hund eine solche Tablette zerkaut, quellen die Bruchstücke wegen dem gelbildenden Polymer auf und die unmittelbare rasche Freisetzung des Wirkstoffs wird dadurch verzögert.

Tiere wie z. B. Hunde können je nach Art unterschiedlich groß sein und ihr Gewicht variiert. Genau auf die jeweilige Größe abgestimmte Dosierungen sind daher kaum im Markt vorhanden, da dies in Herstellung und Vertrieb viel zu aufwendig wäre. Daher werden in der Regel die für kleinere Tiere bestimmten Tabletten auch größeren Tieren verabreicht. In solchen Fällen müssen den größeren Tieren zwei oder mehr Tabletten verabreicht werden. Verwendet man die oben beschriebene übliche Technik der hydrophilen Matrixtabletten, die gelbildende Polymere wie Celluloseether enthalten, quellen die Tabletten im wässrigen Milieu des Gastrointestinaltraktes auf und bilden eine Gelhülle. Wir fanden bei unseren Untersuchungen, dass die Gelschichten solcher Tabletten miteinander verkleben und größere Klumpen im Gastrointestinaltrakt bilden. Die Oberfläche eines solchen Klumpens ist wesentlich kleiner als die Summe der Oberflächen der einzelnen gequollenen Tabletten. Das führt dazu, dass die Freisetzungsrate des Klumpens wesentlich geringer ist als die der einzelnen Tabletten. Die *in vivo* Wirkstofffreisetzung aus den Gelmatrices ist dann nicht mehr reproduzierbar.

Gemäß den Empfehlungen aus dem Stand der Technik sollte bei Retardtabletten für Hunde eine Freisetzungsdauer von bis zu 24 Stunden akzeptabel sein, da die Verweilzeit im Gastrointestinaltrakt (GITT) für etwa 80% der Tabletten mindestens 24 Stunden beträgt. Unerwarteterweise stellten wir aber Folgendes fest: Selbst wenn man nüchternen Hunden Tabletten auf Celluloseether-Basis verabreicht, die *in vitro* > 80% des Wirkstoffs während etwa 12 Stunden freisetzen, findet man in den Faeces Tabletten, die nur teilweise gequollen sind und einen trockenen Kern aufweisen. Dies führt zu einer Verringerung der Bioverfügbarkeit. Die Probleme verstärken sich noch, wenn mehrere Tabletten verabreicht werden, was in der Praxis bei größeren Tieren üblich ist, um die richtige Dosierung zu erreichen. In diesem Fall findet man in den Faeces auch Klumpen von verklebten Tabletten.

McInnes et al., (Pharm Res., Oct 2007) untersuchten zwei verschiedene Matrixtabletten mit unterschiedlichen *in vitro* Freisetzungsraten an gefütterten und nüchternen Hunden. Sie konnten in ihren Untersuchungen keine einfache Korrelation zwischen der *in vitro* und *in vivo* Freisetzung finden. Dies unterstreicht, dass es nicht einfach ist, eine Tablette mit verzögerter Freisetzung zu entwickeln, die einerseits den gesamten Wirkstoff vor Ausscheidung mit den Faeces freisetzt und die andererseits ohne Nachteile zulässt, dass mehr als eine Tablette verabreicht werden kann.

Die vorliegende Erfindung zielt daher darauf ab, eine Tablette mit verzögerter Freisetzung zu entwickeln, die sich im Gastrointestinaltrakt möglichst vollständig auflöst und den Wirkstoff möglichst vollständig innerhalb einer bestimmten Zeit freisetzt, und zwar unabhängig davon, ob das Tier gefressen hat oder nüchtern ist. Dies ist auch deswegen wichtig, weil der Tierhalter oder der Tierarzt das Vertrauen in das Produkt verlieren könnte, wenn er nicht aufgelöste Teile der Tablette in den Faeces findet. Insbesondere wurde angestrebt, ein Matrixsystem zu finden, das vorzugsweise mindestens 80% des gesamten Wirkstoffs in 1 bis 6 Stunden freisetzt und bei dem im wässrigen Milieu des Gastrointestinaltraktes keine Verklumpung auftritt, wenn zwei oder mehr Tabletten verabreicht werden. Schließlich sollte die Herstellung nach Möglichkeit einfach mit üblichen Maschinen der pharmazeutischen Industrie möglich sein.

Die Erfindung betrifft eine feste Arzneimittelzubereitung mit verzögerter Freisetzung, enhaltend:
a. mindestens einen pharmazeutisch aktiven Wirkstoff,
b. 15 bis 40 Gew.-% Polyvinylpyrrolidon und/oder Polyvinylpyrrolidon-Derivat, bei dem es sich um ein Copolymer aus Vinylpyrrolidon und Vinylacetat im Verhältnis 6:4 handelt, wobei es sich bei dem Polyvinylpyrrolidon und/oder Polyvinylpyrrolidon-Derviät um ein Gemisch
   i. eines kurzkettigen Polyvinylpyrrolidons, oder Polyvinylpyrrolidon-Derivats mit einem K-Wert von 17 bis 30 und
   ii. eines längerkettigen Polyvinylpyrrolidons oder Polyvinylpyrrolidon-Derivats mit einem K-Wert von über 40 handelt,
c. mindestens einen Füllstoff.

Als pharmazeutisch aktive Wirkstoffe kommen grundsätzlich alle geeigneten pharmazeutisch wirksamen chemischen Verbindungen in Frage.

Gemäß einer bevorzugten Ausführungsform sind dies anthelmintische Wirkstoffe.

Als eine bevorzugte Gruppe anthelmintischer Wirkstoffe seien Depsipeptide genannt:

Depsipeptide sind den Peptiden ähnlich und unterscheiden sich von diesen darin, dass ein oder mehrere α-Aminosäurebausteine durch α-Hydroxycarbonsäurebausteine ersetzt sind. Erfindungsgemäß bevorzugt eingesetzt werden cyclische Depsipeptide mit 18 bis 24 Ringatomen, insbesondere mit 24 Ringatomen.

Zu den Depsipeptiden mit 18 Ringatomen zählen Verbindungen der allgemeinen Formel (I): 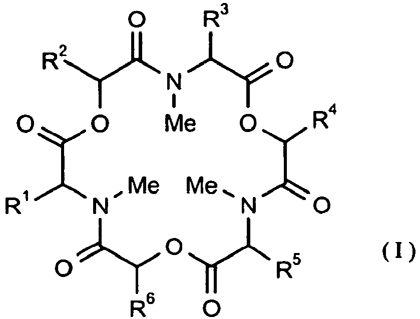 in welcher
R¹, R³ und R⁵ unabhängig voneinander für Wasserstoff geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,

R², R⁴ und R⁶ unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl gegebenenfalls substituiertes Aryl oder Arylalkyl wobei als Substituenten genannt seien Halogen, Hydroxy, Alkyl, Alkoxy, stehen, sowie deren optische Isomere und Racemate.

Bevorzugt sind Verbindungen der Formel (I), 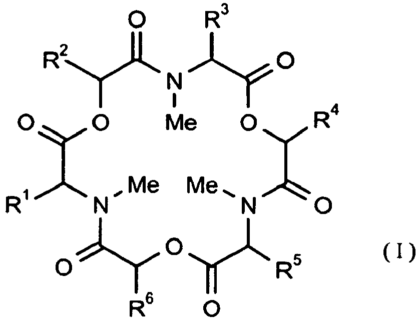 in welcher
R¹, R³ und R⁵ unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl- C₁-C₄-alkyloxy-C₁-C₆-alkyl, insbesondere Benzyloxymethyl, 1-Benzyloxy-ethyl, Mercapto- C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl- C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido- C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)amino-C₁-C₆-alkyl, insbesondere 9-Fluorenyl-methoxycarbonyl-(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl- C₁-C₄-alkyl, insbesondere Cyclopentylmethyl, Cyclohexylmethyl, Cycloheptylmethyl, Phenyl- C₁-C₄-alkyl, insbesondere Phenylmethyl das gegebenenfalls durch Reste aus der Reihe Halogen, insbesondere Fluor, Chlor Brom oder Iod, Hydroxy, C₁-C₄-Alkoxy, insbesondere Methoxy oder Ethoxy, C₁-C₄-Alkyl, insbesondere Methyl, substituiert sein kann,
R², R⁴ und R⁶ unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C1-C6-alkyl, insbesondere Hydroxymethyl, 1-Hydroxyethyl, C₁-C₄-Alkanoyloxy- C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl- C₁-C₄-alkyloxy- C₁-C₆-alkyl, insbesondere Benzyloxymethyl, 1-Benzyloxy-ethyl, Mercapto- C₁-C₆-alkyl, insbesondere Mercaptomethyl, C₁-C₄-Alkylthio-C₁-C₆-alkyl, insbesondere Methylthioethyl, C₁-C₄-Alkylsulfinyl- C₁-C₆-alkyl, insbesondere Methylsulfinylethyl, C₁-C₄-Alkylsulfonyl- C₁-C₆-alkyl, insbesondere Methylsulfonylethyl, Carboxy- C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl- C₁-C₆-alkyl, insbesondere Benzyloxycarbonylmethyl, Carbamoyl- C₁-C₆-alkyl, insbesondere Carbamoylmethyl, Carbamoylethyl, Amino- C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl- C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄-Alkoxy, insbesondere Methoxy oder Ethoxy, C₁-C₄-Alkyl, insbesondere Methyl, substituiert sein kann, sowie deren optische Isomere und Racemate.

### Besonders bevorzugt sind Verbindungen der Formel (I), in welcher

R¹, R³ und R⁵ unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Hydroxy- C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch einen oder mehrere gleiche oder verschiedene der oben angegebenen Reste substituiert sein kann,

R², R⁴ und R⁶ unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C1-C6-alkyl, insbesondere Hydroxymethyl, Aryl- C₁-C₄-alkyloxy-C₁-C₆-alkyl, insbesondere Benzyloxymethyl, 1-Benzyloxyethyl, Carboxy- C₁-C₆-alkyl, insbesondere Carboxymethyl, Carboxyethyl, C₁-C₄-Alkoxycarbonyl- C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonylmethyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl- C₁-C₄-alkyl, insbesondere Cyclopentylmethyl, Cyclohexylmethyl, Cycloheptyl-methyl, Phenyl, Phenyl- C₁-C₄-alkyl, insbesondere Phenylmethyl das gegebenenfalls durch einen oder mehrere gleiche oder verschiedene der oben angegebenen Reste substituiert sein kann, sowie deren optische Isomere und Racemate.

### Ganz besonders bevorzugt sind Verbindungen der Formel (I), in welcher

R¹, R³ und R⁵ unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl C₁-C₈, insbesondere Allyl, C₃-C₇-Cycloalkyl- C₁-C₄-alkyl, insbesondere Cyclohexylmethyl, Phenyl-C₁-C₄-alkyl, insbesondere Phenylmethyl,

R², R⁴ und R⁶ unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈-Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl das gegebenenfalls durch einen oder mehrere gleiche oder verschiedene der oben angegebenen Reste substituiert sein kann, sowie deren optische Isomere und Racemate.

Im einzelnen seien folgende Verbindungen der allgemeinen Formel (I) genannt, in welcher die Reste R¹ bis R⁶ die folgende Bedeutung haben:

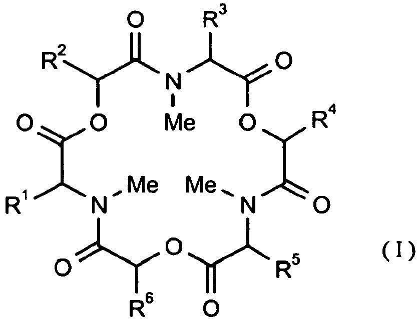

| **R¹** | **R²** | **R³** | **R⁴** | **R⁵** | **R⁶** |
|---|---|---|---|---|---|
| -CHMeCH₂Me | -Cyclohexyl | -CHMeCH₂Me | -Me | -CHMeCH₂Me | -Me |
| -CHMeCH₂Me | -Cyclohexyl | -CHMeCH₂Me | -Me | -CHMeCH₂Me | -Cyclohexyl |
| -CHMeCH₂Me | -CH₂-Phe | -CHMeCH₂Me | -Me | -CHMeCH₂Me | -Me |
| -CHMeCH₂Me | -CH₂-Phe | -CHMeCH₂Me | -Me | -CHMeCH₂Me | -CH₂-Phe |
| -CHMeCH₂Me | -(CH₂)₃-Me | -CHMeCH₂Me | -Me | -CHMeCH₂Me | -Me |
| -CHMeCH₂Me | -(CH₂)₃-Me | -CHMeCH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CH₂-CH=CH₂ | -Me | -CH₂-CH=CH₂ | -Me | -(CH₂)-CH=CH₂ | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -Cyclohexyl | -Me | -Cyclohexyl | -Me | -Cyclohexyl | -Me |
| -CH₂CHMe₂ | -Cyclohexyl | -CH₂CHMe₂ | -Me | -CH₂CHMe₂ | -Cyclohexyl |
| -CH₂CHMe₂ | -Cyclohexyl | -CH₂CHMe₂ | -Me | -CH₂CHMe₂ | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -Cyclohexyl | -Me | -Cyclohexyl | -Me | -Cyclohexyl | -Me |
| -CHMe₂ | -CHMe₂ | -CHMe | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CHMe₂ | -CHMe₂ | -CH₂-Me | -Me |
| -(CH₂)₂-Me | -CHMe₂ | -(CH₂)₂-Me | -Me | -(CH₂)₂-Me | -Me |
| -(CH₂)₂-Me | -CHMe₂ | -(CH₂)₂-Me | -CHMe₂ | -(CH₂)₂-Me | -Me |
| -(CH₂)₃-Me | -CHMe₂ | -(CH₂)₃-Me | -Me | -(CH₂)₃-Me | -Me |
| -(CH₂)₃-Me | -CHMe₂ | -(CH₂)₃-Me | -CHMe₂ | -(CH₂)₃-Me | -Me |
| -CH₂-CH=CH₂ | -CHMe₂ | -CH₂-CH=CH₂ | -Me | -CH₂-CH=CH₂ | -Me |
| -CH₂-CH=CH₂ | -CHMe₂ | -CH₂-CH=CH₂ | -CHMe₂ | -CH₂-CH=CH₂ | -Me |
| -Me | -Me | -CHMeCH₂Me | -Me | -CH₂-Me | -Me |
| -Me | -Me | -CHMeCH₂Me | -Me | -(CH₂)₃-Me | -Me |

| | | | | | |
|---|---|---|---|---|---|
| Me = Methyl; Phe = Phenyl | | | | | |

Weiterhin sei als Depsipeptid die aus EP 0 382 173 bekannte Verbindung PF 1022 der folgenden Formel (IIa) genannt:

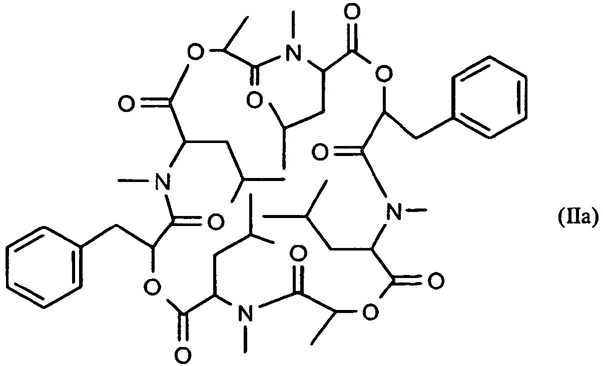

Außerdem seien als Depsipeptide die aus der PCT-Anmeldung WO 93/19053 bekannten Verbindungen genannt.

Insbesondere seien aus WO 93/19053 die Verbindungen der folgenden Formel (IIb) genannt: 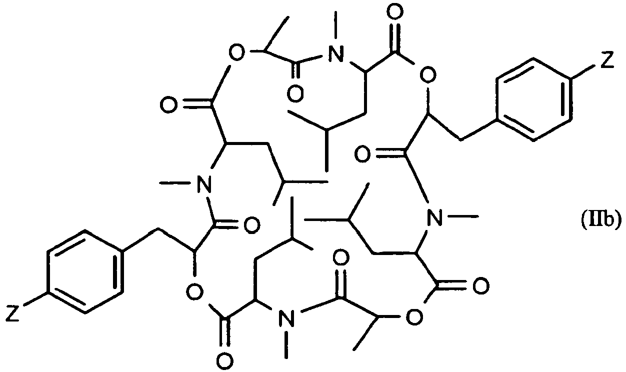 in welcher
Z für N-Morpholinyl, Amino, Mono- oder Dimethylamino steht.

Außerdem seien Verbindungen der folgenden Formel (IIc) genannt: 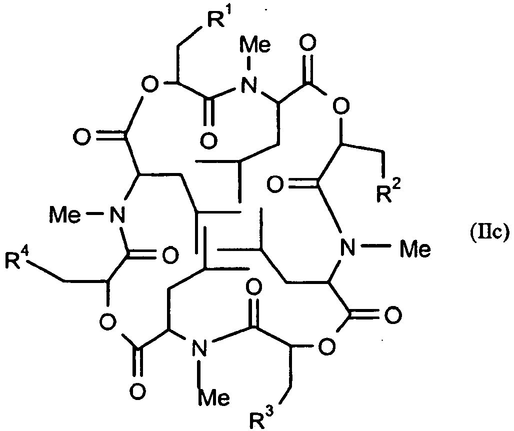 in welcher
R¹, R², R³, R⁴ unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formeln (I) sowie (IIa), (IIb) und (IIc) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren oder in Analogie dazu erhalten werden.

Zu den cyclischen Depsipeptiden mit 24 Ringatomen zählen auch Verbindungen der allgemeinen Formel (IId) 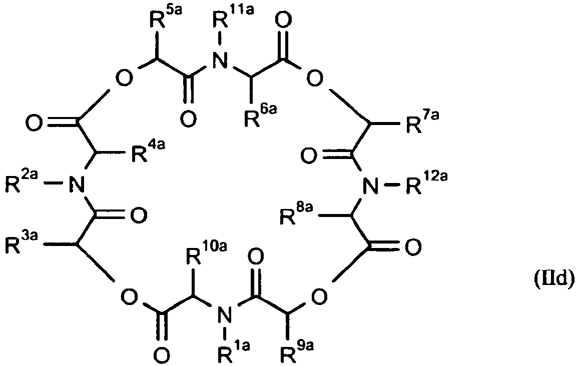 in welcher
R^{1a}, R^{2a} , R^{11a} und R^{12a} unabhängig voneinander für C₁₋₈-Alkyl, C₁₋₈-Halogenalkyl, C₃₋₆-Cycloalkyl, Aralkyl, Aryl stehen,
R^{3a}, R^{5a}, R^{7a}, R^{9a} unabhängig voneinander für Wasserstoff oder geradkettiges oder verzweigtes C₁₋₈-Alkyl steht, das gegebenenfalls durch Hydroxy, C₁₋₄-Alkoxy, Carboxy, 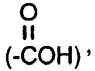 Carboxamid, 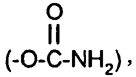 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
R^{4a}, R^{6a}, R^{8a}, R^{10a} unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen sowie deren optische Isomere und Racemate.

### Bevorzugt werden Verbindungen der Formel (IId) eingesetzt, in welcher

R^{1a}, R^{2a}, R^{11a} und R^{12a} unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;

R^{3a} bis R^{10a} die oben angegebene Bedeutung haben.

### Besonders bevorzugt sind Verbindungen der Formel (IId), in welcher

R^{1a}, R^{2a}, R^{11a} und R^{12a} unabhängig voneinander für Methyl, Ethyl, Propyl, Isopropyl oder n-, s-, t-Butyl stehen,
R^{3a}, R^{5a}, R^{7a}, R^{9a} für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
R^{4a}, R^{6a}, R^{8a}, R^{10a} unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die Verbindungen der Formel (IId) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Erfindungsgemäß ganz besonders bevorzugte Depsipeptide sind PF 1022 A (siehe Formel (IIa)) und Emodepside (PF 1022-221, Verbindung der Formel (IIb) worin beide Reste Z für den Morpholinylrest stehen). Der INN Emodepside steht für die Verbindung mit dem systematischen Namen: Cyclo[(R)-lactoyl-*N*-methyl-L-leucyl-(*R*)-3-(*p*-morpholinophenyl)lactoyl-*N*-methyl-L-leucyl-(*R*)-lactoyl-*N-*methyl-L-leucyl-(*R*)-3-(*p*-morpholinophenyl)lactoyl-*N*-methyl-L-leucyl.

Als weitere anthelmintische Wirkstoffe kommen Praziquantel oder Epsiprantel in Frage. Beide sind als Wirkstoffe gegen Endoparasiten lange bekannt (siehe z. B. US 4 661 489 für Epsiprantel und US 4 001 411 für Praziquantel). Praziquantel enthaltende Produkte sind z. B. unter der Bezeichnung Droncit® im Handel erhältlich. Im Rahmen dieser Erfindung ist die Verwendung von Praziquantel bevorzugt.

Gemäß einer besonders bevorzugten Ausführungsform können die Depsipeptide in Kombination mit Praziquantel oder Epsiprantel eingesetzt werden, wobei Praziquantel als Kombinationspartner bevorzugt ist.

Die vorstehend als bevorzugt genannten Depsipeptide sind entsprechend auch in den Kombinationen bevorzugt bzw. besonders bevorzugt.

Gemäß einer ganz besonders bevorzugten Ausführungsform enthalten die erfindungsgemäßen festen Arzneimittelzubereitungen eine Kombination von Praziquantel und PF 1022 A.

Gemäß einer weiteren ganz besonders bevorzugten Ausführungsform enthalten die erfindungsgemäßen festen Arzneimittelzubereitungen eine Kombination von Praziquantel und Emodepside.

Als weitere Wirkstoffe kommen Makrocyclische Lactone in Frage, und zwar insbesondere Avermectine, Dihydroavermectine (Ivermectine) oder Milbemycine. Diese haben anthelmintische Wirkung, zeigen aber auch eine mehr oder weniger starke Wirkung gegen Ectoparasiten, beispielsweise gegen Insekten oder Milben.

Avermectine im engeren Sinne sind insbesondere die acht Avermectin-Komponenten A₁ₐ, A_{1b}, A₂ₐ, A_{2b}, B₁ₐ, B_{1b}, B₂ₐ und B_{2b}. In der Praxis wird beispielsweise das mit Abamectin bezeichnete Gemisch eingesetzt, das im wesentlichen die Avermectine B₁ enthält. Weiterhin werden z. B. Doramectin und Selamectin zu den Avermectinen gerechnet.

Das Hydrierungsprodukt des Abamectins wird als Ivermectin bezeichnet, es handelt sich entsprechend um die 22,23-Dihydroavermectine B₁.

Als Milbemycine seien genannt Milbemycin B41 D, Nemadectin, Moxidectin.

Gemäß einer weiteren ganz besonders bevorzugten Ausführungsform enthalten die erfindungsgemäßen festen Arzneimittelzubereitungen eine Kombination von Praziquantel, Emodepside und einem der oben genannten Makrocyclischen Lactone. Von diesen ganz besonders bevorzugt ist in dieser Ausführungsform Ivermectin.

Als eine weitere Gruppe von Wirkstoffen kommen Analgetika in Frage wie z. B. Nichtopioid-Analgetika oder Opiod-Analgetika. Als Nichtopioid-Analgetika seien beispielsweise Meloxicam, Carprofen und Metamizol genannt. Als Opioid-Analgetika seien beispielsweise genannt Bupremorphin und Fentanyl.

Als bevorzugtes Beispiel sei Metamizol (N-Methyl-N-(2,3-dimethyl-5-oxo-1-phenyl-3-pyrazolin-4-yl)aminomethansulfonsäure, auch als Dipyrone bezeichnet) genannt, das in der Regel in Form seines Natriumsalzes eingesetzt wird. Andere pharmazeutisch verträgliche Salze können ebenfalls verwendet werden. Metamizol ist genau genommen als Prodrug anzusehen, das vier Hauptmetaboliten hat. Zwei davon sind wirksam, und zwar vor allem 4-N-Methylaminoantipyrin (4-MAA) und Aminoantipyrin (4-AA).

Weiterhin als Arzneimittelwirkstoffe einsetzbar sind pharmakologisch verträgliche Phosphonsäurederivate, wobei es sich üblicherweise um organische Verbindungen handelt, die sich als Stoffwechselstimulantien und Tonika insbesondere für Nutz- und Haustiere eignen. Als bevorzugte Beispiele seien die bereits lange bekannten Verbindungen Toldimfos und insbesondere Butaphosphan (z. B. verwendet in dem Produkt Catosal®) genannt, die unter anderem der Ergänzung von Mineralstoffen (Phosphor) dienen.

Wirkstoffe können je nach Struktur in stereoisomeren Formen oder als Stereoisomerengemische vorliegen, z.B. als Enantiomere oder Racemate. Sowohl die Stereoisomerengemische als auch die reinen Stereoisomeren können erfindungsgemäß verwendet werden.

Weiterhin können gegebenenfalls verwendet werden: Salze der Wirkstoffe mit pharmazeutisch annehmbaren Säuren oder Basen und auch Solvate, insbesondere Hydrate, der Wirkstoffe oder ihrer Salze.

Gemäß einer bevorzugten Ausführungsform handelt es sich bei den erfindungsgemäßen Zubereitungen um Tabletten.

Die Zubereitungen enthalten retardierende Polymere, bei denen es sich um wasserquellbare Polymere handelt. Da die wasserquellbaren Polymeren in Gegenwart von Wasser Gele bilden, kann man sie auch als "gelbildende Polymere" bezeichnen. Erfindungsgemäß werden als wasserquellbare Polymere Polyvinylpyrrolidone oder deren Derivate eingesetzt, auch der Einsatz von Mischungen von Polyinylpyrrolidonen und Polyvinylpyrrolidon-Derivaten ist denkbar. Besonders bevorzugt ist jedoch der Einsatz von Polyvinylpyrrolidonen.

Denkbar ist auch der Einsatz von Polyvinylpyrrolidonen in Kombination mit anderen geeigneten Polymeren, als Beispiel sei Kollidon® SR der Fa. BASF genannt. Dabei handelt es sich um eine Mischung, die sprühgetrocknetes Polyvinylacetat (mit einem gewichtsmittleren Molekulargewicht von etwa 450 000) und lösliches Polyvinylpyrrolidon (Povidon K 30) im Verhältnis 8 : 2 enthält.

Als Beispiel für ein geeignetes Polyvinylpyrrolidon-Derivat sei Copovidon (z. B. Kollidon VA 64 der Fa. BASF) genannt. Dabei handelt es sich um ein Copolymer aus Vinylpyrrolidon und Vinylacetat im Verhältnis 6:4.

Polyvinylpyrrolidone (Povidone, PVP) sind im Handel erhältliche hydrophile Polymere, die sich für die Verwendung in festen Arzneimittelzubereitungen mit verzögerter Freisetzung eignen. Verschiedene PVP-Arten sind im Handel erhältlich. PVP mit niedrigerem Molekulargewicht werden üblicherweise als Bindemittel für Tabletten eingesetzt. Im wässrigen Milieu quellen PVP und erodieren. Es zeigte sich aber, dass PVP-haltige Tabletten keine klebrige Gelschicht bilden, wie z. B. Celluloseether. Gemäß unseren *in vitro* Versuchen verkleben Tabletten enthaltend PVP auch im wässrigen Milieu nicht. Bei Verabreichung mehrerer Tabletten ist das Risiko einer Klumpenbildung im Gastrointestinaltrakt gering. Indem man PVP mit verschiedenen Molekulargewichten verwendet, kann man die Freisetzungskinetik in einem definierten Bereich variieren.

Die eingesetzten Polyvinylpyrrolidone oder Polyvinylpyrrolidon-Derivate sind vorzugsweise wasserlöslich. Hierbei handelt es sich in der Regel um lineare nicht quervernetzte Polyvinylpyrrolidone oder Polyvinylpyrrolidon-Derivate.

Die Polyvinylpyrrolidone oder Polyvinylpyrrolidon-Derivate weisen einen K-Wert von mindestens 17 auf.

Der K-Wert der Polyvinylpyrrolidone oder Polyvinylpyrrolidon-Derivate steht in Beziehung zur Viskosität und zum Molekulargewicht und kann nach an sich bekannten Methoden bestimmt werden. Im Zweifel werden die Angaben zum K-Wert aus der European Pharmacopeia (Ph. Eur.) verwendet.

Es werden Polyvinylpyrrolidone und/oder Polyvinylpyrrolidon-Derivate mit einem K-Wert von 17 bis 90, besonders bevorzugt 25 bis 90 eingesetzt.

Die fertige Formulierung enthält 15 bis 40 Gew.-%*ₜ* bevorzugt 25 bis 35 Gew.-% an Polyvinylpyrrolidon oder Polyvinylpyrrolidon-Derivat oder an deren Gemisch.

Gemäß der Erfindung werden ein Polyvinylpyrrolidon und/oder Polyvinyl- und/oder Polyvinylpyrrolidon-Derivat mit einer geringeren Kettenlänge und eines mit einer höheren Kettenlänge eingesetzt. Auf diese Weise lässt sich die Freisetzungscharakteristik besonders gut einstellen, da mit kurzkettigen Polyvinylpyrrolidonen oder Polyvinylpyrrolidon-Derivaten eine relativ schnelle Freisetzung erreicht wird, während längerkettige Polyvinylpyrrolidone oder Polyvinylpyrrolidon-Derivate zu einer langsameren Freisetzung führen. Üblicherweise kann das Verhältnis von längerkettigem zu kurzkettigem Polyvinylpyrrolidon oder Polyvinylpyrrolidon-Derivat in einem Bereich von 1:10 Gewichtsteilen bis hin zum ausschließlichen Einsatz des längerkettigen Polyvinylpyrrolidons oder Polyvinylpyrrolidon-Derivats variieren. Das genaue Verhältnis sollte entsprechend dem Diffusionsverhalten des verwendeten Wirkstoffs eingestellt werden. Gut wasserlösliche Wirkstoffe wie z. B. Metamizol diffundieren leicht aus dem Gel heraus. In diesem Fall kann eine geeignete Freisetzungskinetik ohne kurzkettiges Polyvinylpyrrolidon oder Polyvinylpyrrolidon-Derivat oder mit relativ geringen Mengen davon erreicht werden. Das Gewichtsverhältnis von langkettigem zu kurzkettigem Polyvinylpyrrolidon oder Polyvinylpyrrolidon-Derivat liegt daher gemäß einer bevorzugten Ausführungsform im Bereich von mindestens 5:1 bis zum ausschließlichen Einsatz des langkettigen Polyvinylpyrrolidons, bevorzugt liegt das Gewichtsverhältnis bei mindestens 10:1.

Weniger gut wasserlösliche Wirkstoffe wie Emodepside oder Praziquantel diffundieren langsamer aus dem Gel heraus und werden im Wesentlichen mit der Erosion des Gels freigesetzt. Hier ist daher ein höherer Anteil an kurzkettigem Polyvinylpyrrolidon oder Polyvinylpyrrolidon-Derivat empfehlenswert, um die gewünschte Freisetzungskinetik zu erreichen. Gemäß einer weiteren bevorzugten Ausführungsform liegt daher das Gewichtsverhältnis von langkettigem zu kurzkettigem Polyvinylpyrrolidon oder Polyvinylpyrrolidon-Derivat im Bereich von 1:1 bis 5:1, bevorzugt 2:1 bis 4:1.

Das kurzkettige Polyvinylpyrrolidon oder Polyvinylpyrrolidon-Derivat hat einen K-Wert von 17 bis 30, bevorzugt etwa 25.

Das längerkettige Polyvinylpyrrolidon oder Polyvinylpyrrolidon-Derivat hat einen K-Wert von über 40, vorzugsweise 60 bis 120, besonders bevorzugt etwa 90.

Details bezüglich der oben genannten Polyvinylpyrrolidone, Polyvinylpyrrolidon-Derivate und bestimmter Mischungen können dem folgenden Buch entnommen werden: V. Bühler, "Kollidon, Polyvinylpyrrolidone for the pharmaceutical industry,", 9 th revised edition, BASF Pharma Ingredients, Germany, 2008.

Zur Einstellung der Freisetzungsrate der erfindungsgemäßen Zubereitungen können wasserlösliche Hilfsstoffe eingesetzt werden, wie z. B. Polyethylenglykol, Lactose (insbesondere als Lactose-Monohydrat) oder mehrwertige Alkohole, z. B. Mannitol, Sorbitol, Xylitol oder Mischungen der vorstehend genannten Hilfstoffe; diese Hilfsstoffe sind gegebenenfalls in Mengen von üblicherweise 1 bis 20 % (m/m), bevorzugt 5 bis 15% (m/m) enthalten.

Bevorzugt kann durch Einarbeitung von Sprengmitteln, wie z.B. Stärke, quervernetzte Natrium-Carboxymethylcellulose (Croscarmellose-Natrium), Natriumstärkeglycolat, vernetztes Polyvinylpyrrolidon (Crospovidone, wie z. B. ^{®}Kollidon CL), die Freisetzungscharakteristik der erfindungsgemäßen Zubereitungen weiter variiert werden. Die Verwendung der vorstehend genannten wasserlöslichen Hilfsstoffe ist dann nicht unbedingt erforderlich. Ein bevorzugtes Sprengmittel ist Croscarmellose-Natrium, Ein weiteres bevorzugtes Sprengmittel ist Crospovidone. Sofern Sprengmittel verwendet werden, sind diese üblicherweise in Mengen bis 5% (m/m), bevorzugt 0,1 bis 3% (m/m), besonders bevorzugt 0,5 bis 1,5 % (m/m) enthalten.

Insgesamt lässt sich durch die oben beschriebenen Maßnahmen eine gut reproduzierbare Bioverfügbarkeit erreichen und das Risiko in den Faeces unzersetzte oder teilweise zersetzte Tabletten zu finden ist sehr gering.

Als Füllstoffe kommen für feste Zubereitungen (z. B. Tabletten) übliche Füllstoffe in Frage, wie zum Beispiel Carbonate wie Calciumcarbonat, Hydrogencarbonate, Kochsalz, Aluminiumoxide, Kieselsäuren, Tonerden, Phosphate (vor allem Calciumphosphate) oder organische Füllstoffe wie Lactose oder mikrokristalline Cellulose. Bevorzugt wird wasserfreies Calciumhydrogenphosphat eingesetzt. Ebenfalls bevorzugt ist mikrokristalline Cellulose. Es können auch verschiedene Füllstoffe miteinander kombiniert werden. Die Gesamtmenge an Füllstoff(en) liegt üblicherweise bei 5 bis 80% (m/m), bevorzugt 10 bis 70 % (m/m), besonders bevorzugt 20 bis 60 % (m/m).

Weiterhin können die erfindungsgemäßen festen Arzneimittelzubereitungen neben dem oder den Wirkstoffen und den anderen vorstehend genannten Inhaltsstoffen auch Hilfsstoffe enthalten, wie zum Beispiel: Gleitmittel, z.B. kolloidales Siliciumdioxid wie Aerosil^{®}, hydrierte pflanzliche Öle, Stearinsäure, Talkum oder deren Mischungen sind gegebenenfalls in Mengen von üblicherweise 0,1 bis 2% (m/m), bevorzugt 0,5 bis 1% (m/m) enthalten. Schmiermittel wie z.B. Magnesiumstearat sind gegebenenfalls in Mengen von üblicherweise 0,3 bis 2% (m/m), bevorzugt 0,5 bis 1,5 % (m/m) enthalten.

Zur Verbesserung der Palatabilität werden gemäß einer bevorzugten Ausführungsform Aromen und/oder Geschmacksstoffe zugegeben.

Als Fleischaroma sind Trockenleberpulver aus Rind, Geflügel, Schaf oder Schwein bevorzugt aus Geflügel und Schwein, sowie andere Aromenzubereitungen geeignet. Gemäß einer bevorzugten Ausführungsform sind Geschmacks- bzw. Aromastoffe geeignet, bei denen es sich um Mischungen aus Proteinen, Fetten und Kohlenhydraten handelt, die speziell aufgearbeitet werden, insbesondere sei genannt Artificial Beef Flavor^{®} der Firma Pharma Chemie (Syracuse, Nebraska, USA). Artificial Beef Flavor^{®} ist ein Schweineleberextrakt, dem weitere Proteine zugesetzt werden. Gemäß einer weiteren bevorzugten Ausführungsform kann auch Trockenleberpulver eingesetzt werden. In den erfindungsgemäßen Arzneimittelformulierungen werden die Geschmacks- bzw. Aromastoffe in einer Menge von 1 - 40 Gew.-%, bezogen auf das Gesamtgewicht der fertigen Formulierung, vorzugsweise 5-30 Gew.-%, insbesondere 10-25 Gew.-% eingesetzt. Die Prozentangaben sind dabei Gewichtsprozent der fertigen Formulierung.

Die erfindungsgemäßen Zubereitungen können z. B. hergestellt werden, indem man die Bestandteile mischt oder granuliert und dann zu Tabletten verpresst. Bevorzugt sind Nassgranulierverfahren. Vorzugsweise werden Aroma bzw. Geschmackstoffe, Sprengmittel, Gleit- und Schmiermittel nach der Granulation zugemischt und die Mischung anschließend tablettiert.

Die *in vitro*-Freisetzung der erfindungsgemäßen Zubereitungen kann in herkömmlichen Freisetzungsapparaturen bestimmt werden, und zwar mit dem "Paddle-Test" gemäß US Pharmacopeia (USP) unter Sink-Bedingungen. "Sink-Bedingungen" ist ein in der Pharmazie üblicher Begriff, er beinhaltet, dass Art und Menge des verwendeten Freisetzungsmediurns so gewählt werden, dass sich die dreifache Menge des betreffenden Wirkstoffs darin lösen würde. Das maximale Volumen an Freisetzungsmedium beträgt 900 ml. Das Medium enthält als wesentliche Komponente Wasser, dem ggf. zur Verbesserung der Löslichkeit ein Tensid zugesetzt wird. Mit üblichen Puffern wird der pH eingestellt, bei dem der betreffende Wirkstoff am stabilsten ist. Ziel ist es bei den erfindungsgemäßen Formulierungen eine mindestens 80 %ige, bevorzugt mindestens 85%ige, insbesondere mindestens 90%ige *in vitro* Freisetzung des Wirkstoffs in 1 bis 6 Stunden, bevorzugt in 1 bis 5 Stunden, besonders bevorzugt in 1,5 bis 5 Stunden zu erreichen. Gemessen wird bei 37°C und 75 U/min. Im Fall der Freisetzung von Depsipeptiden, wie Emodepsid, aus Depsipeptid-haltigen Formulierungen hat das Freisetzungsmedium pH 3.0 (Dinatriumhydrogenphosphat-Dihydrat/Citronensäure-Monohydrat-Puffer) und es wurden 0,5 % Natriumlaurylsulfat zugesetzt. Für Formulierungen die pro Einheit bis zu 10 mg Emodepsid enthalten, ist das Volumen 500 ml. Für Einheiten (z. B. Tabletten) mit höherem Emodepsid-Gehalt müssen die Sinkbedingungen eingehalten werden. So werden für 30mg-Einheiten 900 ml Medium benötigt.

Im Fall von Zubereitungen mit Metamizol sind die Bedingungen zur Bestimmung der *in vitro* Freisetzung wie folgt: pH 6,8 (Phosphatpuffer, Standardfreisetzungsmedium nach USP), 900 ml.

Die erfindungsgemäßen Zubereitungen eignen sich zur Anwendung bei Menschen und in der Tierhaltung und Tierzucht bei Nutz-, Zucht-, Zoo-, Labor-, Versuchs- und Hobbytieren.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße.

Zu Labor- und Versuchstieren gehören Mäuse, Ratten, Meerschweinchen, Goldhamster, Hunde und Katzen.

Zu den Hobbytieren gehören Hunde und Katzen. Besonders bevorzugt ist die Anwendung bei Katzen und insbesondere Hunden.

Gemäß einer bevorzugten Ausführungsform enthalten die Zubereitungen anthelmintische Wirkstoffe , wie weiter oben beschrieben. Sie eignen sie sich dann zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Je nach eingesetztem Wirkstoff sind sie dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen:

Aus der Ordnung der Pseudophyllidea z.B.: Diphyllobothrium spp., Spirometra spp., Schistocephalus spp., Ligula spp., Bothridium spp., Diphlogonoporus spp.

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Omithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp.

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Oxyurida z.B.: Oxyuris spp., Enterobius spp., Passalurus spp., Syphacia spp., Aspiculuris spp., Heterakis spp.

Aus der Ordnung der Ascaridia z.B.: Ascaris spp., Toxascaris spp., Toxocara spp., Parascaris spp., Anisakis spp., Ascaridia spp.

Aus der Ordnung der Spirurida z.B.: Gnathostoma spp., Physaloptera spp., Thelazia spp., Gongylonema spp., Habronema spp., Parabronema spp., Draschia spp., Dracunculus spp.

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.

Aus der Ordnung der Gigantorhynchida z.B.: Filicollis spp., Moniliformis spp., Macracanthorhynchus spp., Prosthenorchis spp.

Besonders bevorzugt ist die Anwendung der erfindungsgemäßen Mittel gegen: *Toxocara cati, Toxascaris leonina, Ancylostoma tubaeforme, Dipylidium caninum, Taenia taeniaeformis* und *Echinococcus multilocularis.*

Auch bei anderen Wirkstoffen eignen sich die Zubereitungen grundsätzlich zur Behandlung der Indikationen, für welche sich die jeweiligen Wirkstoffe bekanntermaßen per se eignen.

Analgetika wie Metamizol können z. B. für die Behandlung leichter sowie mittelschwerer bis schwerer Schmerzzustände eingesetzt werden, wie beispielsweise: Posttraumatischer Schmerz (z.B. stumpfes Trauma, Distorsionen), perioperativer Schmerz, postoperativer Schmerz, Tumorschmerz, osteoarthritischer Schmerz, Tendopathien, abdominaler Weichteilschmerz, geriatrischer Zahnschmerz.

Die Anwendung kann sowohl prophylaktisch als auch therapeutisch erfolgen.

### Beispiele

### A. Formulierungsbeispiele

Zur Herstellung der folgenden Beispiele werden wasserfreies Calciumhydrogenphosphat, Povidone 90 (sowie gegebenenfalls Copovidon 64), und ein Teil der Gesamtmenge an Povidone 25 und mikrokristalline Cellulose gemischt, dann werden Emodepsid und Praziquantel eingemischt. Die Mischung wird mit einer wässrigen Lösung des zweiten Teils an Povidone 25 granuliert und in einem Wirbelschichtgranulator bei Temperaturen unterhalb von 110°C getrocknet. Das Granulat wird gesiebt und mit Artificial Beef Flavour, Natrium-Croscarmellose, wasserfreiem kolloidalem Siliciumdioxid und Magnesiumstearat gemischt.

Das so erhaltene Material kann zu Tabletten gepresst werden.

### Beispiel 1

| | |
|---|---|
| 3,00 mg | Emodepside |
| 15,00 mg | Praziquantel |
| 19,20 mg | wasserfreies Calciumhydrogenphosphat |
| 37,80 mg | mikrokristalline Cellulose |
| 31,50 mg | Artificial beef flavour (PC 0125, Pharma Chemie Inc., Syracuse/USA) |
| 36,00 mg | Povidone 90 (Polyvinylpyrrolidon mit K-Wert 90) |
| 12,90 mg | Povidone 25 (Polyvinylpyrrolidon mit K-Wert 25) |
| 0,90 mg | wasserfreies kolloidales Siliciumdioxid |
| 1,20 mg | quervernetzte Natrium-Carboxymethylcellulose (Natrium-Croscarmellose) |
| 1,50 mg | Magnesiumstearat |

### Beispiel 2

| | |
|---|---|
| 10,00 mg | Emodepside |
| 50,00 mg | Praziquantel |
| 64,00 mg | wasserfreies Calciumhydrogenphosphat |
| 126,00 mg | mikrokristalline Cellulose |
| 105,00 mg | Artificial beef flavour (PC 0125, Pharma Chemie Inc., Syracuse/USA) |
| 120,00 mg | Povidone 90 (Polyvinylpyrrolidon mit K-Wert 90) |
| 43,00 mg | Povidone 25 (Polyvinylpyrrolidon mit K-Wert 25) |
| 3,00 mg | wasserfreies kolloidales Siliciumdioxid |
| 4,00 mg | quervernetzte Natrium-Carboxymethylcellulose (Natrium-Crosearmellose) |
| 5,00 mg | Magnesiumstearat |

### Beispiel 3

| | |
|---|---|
| 10,00 mg | Emodepside |
| 50,00 mg | Praziquantel |
| 64,00 mg | wasserfreies Calciumhydrogenphosphat |
| 126,00 mg | mikrokristalline Cellulose |
| 37,50 mg | Artificial beef flavour (PC 0125, Pharma Chemie Inc., Syracuse/USA) |
| 40,00 mg | Povidone 90 (Polyvinylpyrrolidon mit K-Wert 90) |
| 42,00 mg | Povidone 25 (Polyvinylpyrrolidon mit K-Wert 25) |
| 2,00 mg | wasserfreies kolloidales Siliciumdioxid |
| 4,00 mg | quervernetzte Natrium-Carboxymethylcellulose (Natrium-Croscarmellose®) |
| 3,75 mg | Magnesiumstearat |

### Beispiel 4

| | |
|---|---|
| 500,00 mg | Metamizol-Natrium |
| 300,00 mg | mikrokristalline Cellulose |
| 300,00 mg | Povidone 90 (Polyvinylpyrrolidon mit K-Wert 90) |
| 10,00 mg | wasserfreies kolloidales Siliciumdioxid |
| 10,00 mg | Magnesiumstearat |

### Beispiel 5

| | |
|---|---|
| 1000,00 mg | Metamizol-Natrium |
| 600,00 mg | mikrokristalline Cellulose |
| 600,00 mg | Povidone 90 (Polyvinylpyrrolidon mit K-Wert 90) |
| 20,00 mg | wasserfreies kolloidales Siliciumdioxid |
| 20,00 mg | Magnesiumstearat |
| 278,00 mg | Trockenlebermehl |

### Beispiel 6

| | |
|---|---|
| 10,00 mg | Emodepside |
| 50,00 mg | Praziquantel |
| 64,00 mg | wasserfreies Calciumhydrogenphosphat |
| 126,00 mg | mikrokristalline Cellulose |
| 105,00 mg | Artificial beef flavour (PC 0125, Pharma Chemie Inc., Syracuse/USA) |
| 90,00 mg | Povidone 90 (Polyvinylpyrrolidon mit K-Wert 90) |
| 30,00 mg | Copovidon 64 (Kollidon® VA 64 der Fa. BASF, Copolymerisat von Vinylpyrrolidon und Vinylacetat im Verhältnis 6 : 4) |
| 43,00 mg | Povidone 25 (Polyvinylpyrrolidon mit K-Wert 25) |
| 3,00 mg | wasserfreies kolloidales Siliciumdioxid |
| 4,00 mg | quervernetzte Natrium-Carboxymethylcellulose (Natrium-Croscarmellose) |
| 5,00 mg | Magnesiumstearat |

### Beispiel 7

| | |
|---|---|
| 10,00 mg | Emodepside |
| 50,00 mg | Praziquantel |
| 64,00 mg | wasserfreies Calciumhydrogenphosphat |
| 126,00 mg | mikrokristalline Cellulose |
| 105,00 mg | Artificial beef flavour (PC 0125, Pharma Chemie Inc., Syracuse/USA) |
| 30,00 mg | Povidone 90 (Polyvinylpyrrolidon mit K-Wert 90) |
| 90,00 mg | Copovidon 64 (Kollidon® VA 64 der Fa. BASF, Copolymerisat von Vinylpyrrolidon und Vinylacetat im Verhältnis 6 : 4) |
| 43,00 mg | Povidone 25 (Polyvinylpyrrolidon mit K-Wert 25) |
| 3,00 mg | wasserfreies kolloidales Siliciumdioxid |
| 4,00 mg | quervernetzte Natrium-Carboxymethylcellulose (Natrium-Croscarmellose) |
| 5,00 mg | Magnesiumstearat |

### Vergleichsbeispiel (nicht erfindungsgemäße Formulierung)

| | |
|---|---|
| 5,00 mg | Emodepside |
| 50,00 mg | Praziquantel |
| 30,00 mg | wasserfreies Calciumhydrogenphosphat |
| 63,00 mg | mikrokristalline Cellulose |
| 35,00 mg | Artificial beef flavour |
| 92,00 mg | Hydroxypropylcellulose -M (HPC-M, Fa. Nisso, Japan) |
| 1,00 mg | wasserfreies kolloidales Siliciumdioxid |
| 3,00 mg | Magnesiumstearat |

Nach der Rezeptur des Vergleichsbeispiels hergestellte Tabletten erreichten in dem USP-Freisetzungstest eine Freisetzung von > 80% des Wirkstoffs innerhalb von 12 Stunden. Bei Versuchen mit Hunden waren nicht vollständig aufgelöste Tabletten bzw. Tablettenreste im Kot zu finden. Bei Verabreichung mehrerer Tabletten kam es zum Verkleben und Verklumpen.

### In vitro Freisetzung

Die *in vitro*-Freisetzung der erfindungsgemäßen Zubereitungen wurde mit dem "Paddle-Test" gemäß US Pharmacopeia (USP) unter Sink-Bedingungen bestimmt.

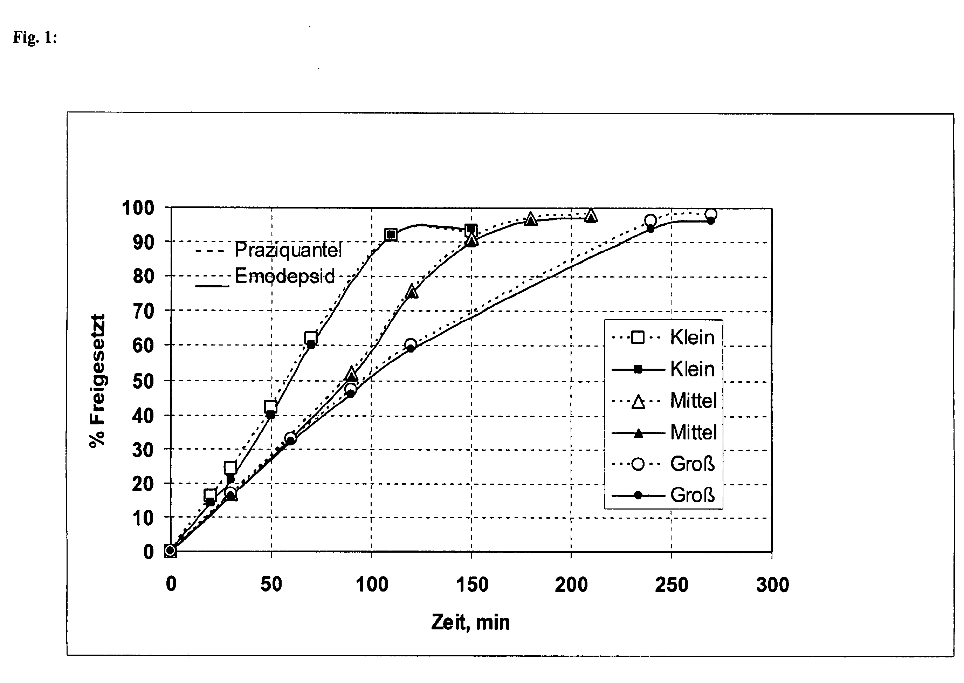

Fig. 1 zeigt die Ergebnisse für verschiedene Emodepsid/Praziquantel-Tabletten:
"Klein" steht für die Tabletten gemäß Beispiel 1
"Mittel" steht für Tabletten gemäß Beispiel 2
"Groß" steht für größere Tabletten mit 30 mg Emodepsid und 150 mg Praziquantel pro Tablette. Die Tabletten haben dieselbe prozentuale Zusammensetzung wie diejenigen der Beispiele 1 und 2.

Meßbedingungen: 37°C, 75 U/min, wäßriges Medium mit pH 3.0 (Dinatriumhydrogenphosphat-Dihydrat/Citronensäure-Monohydrat-Puffer), 0,5 % Natriumlaurylsulfat. Für die kleinen und mittleren Tabletten wurden 500 ml Freisetzungsmedium verwendet für die großen 900 ml.

Fig. 1 zeigt, dass eine über 90 %ige Freisetzung bei allen Tabletten nach 1 bis 5 Stunden erreicht wird.

### A. Biologische Beispiele

### I. Pharmakokinetische Untersuchungen

Das zu untersuchende Arzneimittel wurde nüchternen Hunden verabreicht. Der Plasmaspiegel des Wirkstoffs bzw. der Wirkstoffe wurde zu verschiedenen Zeitpunkten bestimmt.

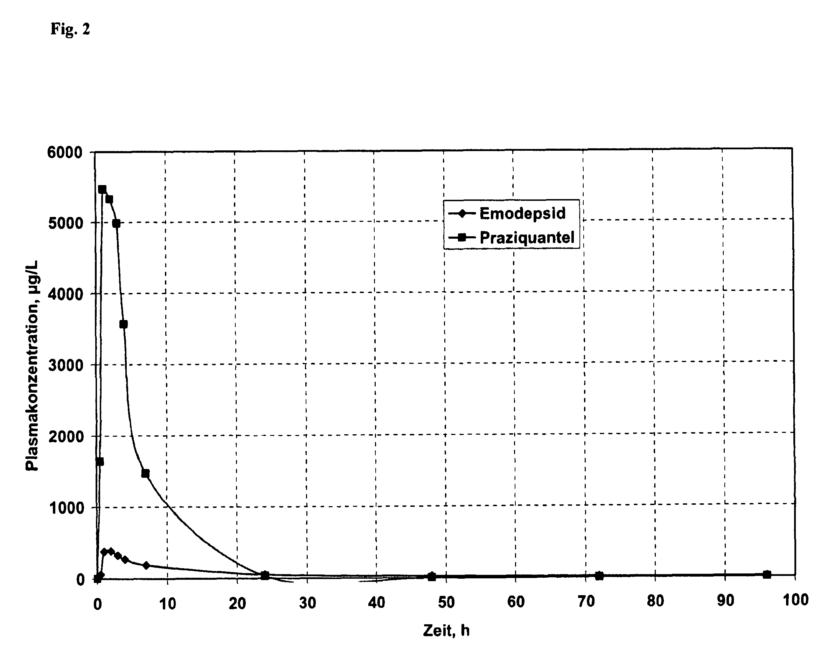

Fig. 2 zeigt die Ergebnisse nach Gabe einer Tablette gemäß Beispiel 2. Man erkennt bei sowohl bei Praziquantel als auch inbesondere bei Emodepsid ein deutlich verzögertes Absinken der Plasmakonzentration.

### II. Vergleich einer Metamizol-Tablettenformulierung mit einer handelsüblichen Lösung zur intravenösen Applikation

6 Hunde (Körpergewicht von 9,9 - 11,1 kg) wurden in zwei Gruppen zu je 3 Hunden aufgeteilt. Einer Gruppe wurde Metamizol in Form der handelsüblichen Injektionsformulierung Metapyrin® in einer Dosis von 500 mg/Hund intravenös verabreicht. Der zweiten Gruppe wurde Metamizol in Form der Formulierung aus Beispiel 4 ebenfalls in der Dosierung 500 mg/Hund oral verabreicht. Die Hunde waren bei der Applikation nüchtern.

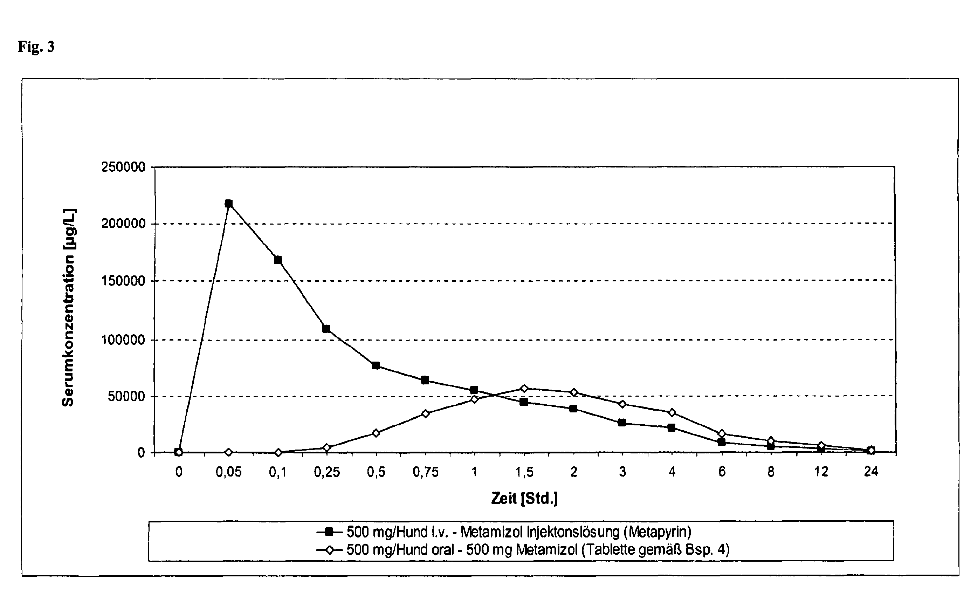

Fig. 3 zeigt die Plasmaspiegel an Metamizol nach Applikation, wobei es sich bei der angegebenen Metamizolkonzentration [4-MAA + 4-AA] um einen errechneten Wert handelt, der aus der Summe der Serumkonzentrationen der beiden aktiven Hauptmetaboliten 4-MAA und 4-AA unter Berücksichtigung der Molmasse dieser beiden Metaboliten bestimmt wird.

### III. Plasmakonzentrationen nach oraler Verabreichung von ein oder zwei Metamizol-Tabletten

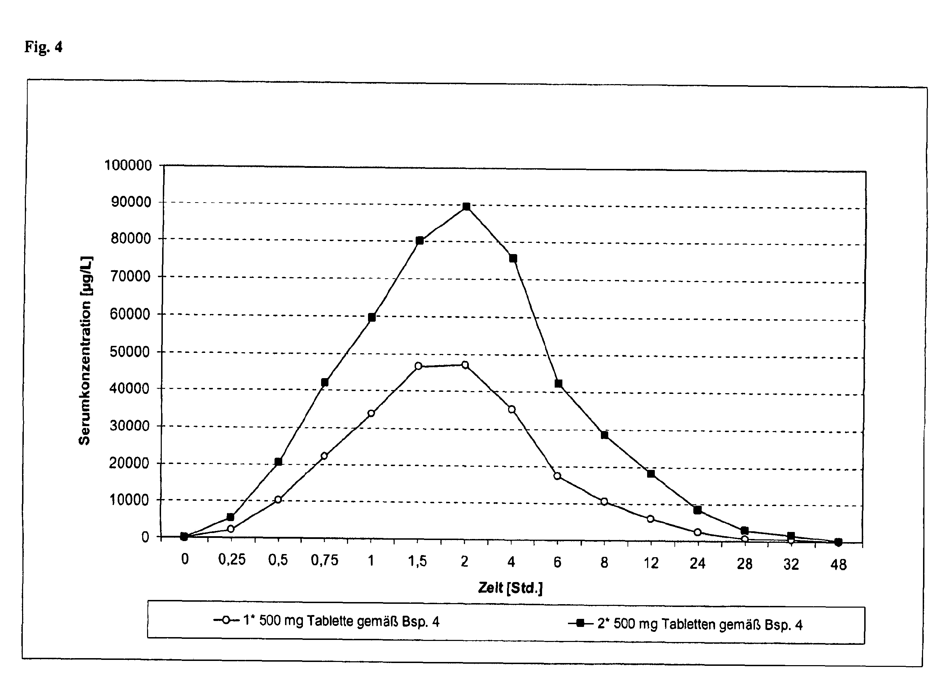

Fig. 4 zeigt die mittlere Metamizol-Konzentration [4-MAA + 4-AA] im Serum nüchterner Hunde nach oraler Gabe von einer bzw. zwei Metamizol-Tabletten gemäß Bsp. 4. Man erkennt, dass die Plasmaspiegel recht gut mit der verabreichten Dosis korrelieren.

## Patentansprüche

1. Feste Arzneimittelzubereitung mit verzögerter Freisetzung, enhaltend:
a. mindestens einen pharmazeutisch aktiven Wirkstoff,
b. 15 bis 40 Gew.-% Polyvinylpyrrolidon und/oder Polyvinylpyrrolidon-Derivat, bei dem es sich um ein Copolymer aus Vinylpyrrolidon und Vinylacetat im Verhältnis 6:4 handelt, wobei es sich bei dem Polyvinylpyrrolidon und/oder Polyvinylpyrrolidon-Derviat um ein Gemisch
i. eines kurzkettigen Polyvinylpyrrolidons oder Polyvinylpyrrolidon-Derivats mit einem K-Wert von 17 bis 30 und
ii. eines längerkettigen Polyvinylpyrrolidons oder Polyvinylpyrrolidon-Derivats mit einem K-Wert von über 40 handelt,
c. mindestens einen Füllstoff.

2. Feste Arzneimittelzubereitung gemäß einem der vorstehenden Ansprüche, zusätzlich enthaltend ein Sprengmittel.

3. Feste Arzneimittelzubereitung gemäß Anspruch 2, enthaltend das Sprengmittel in Mengen bis 5% (m/m).

4. Feste Arzneimittelzubereitung gemäß einem der vorstehenden Ansprüche, enthaltend als Depsipeptid eine Verbindung der allgemeinen Formel (I): 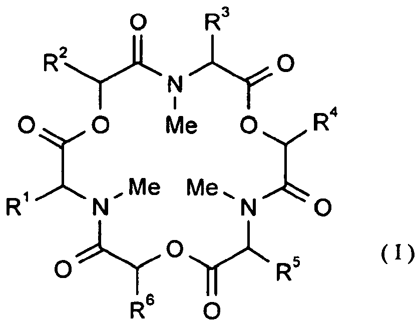 in welcher
R¹, R³ und R⁵ unabhängig voneinander für Wasserstoff geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,
R², R⁴ und R⁶ unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl gegebenenfalls substituiertes Aryl oder Arylalkyl wobei als Substituenten genannt seien Halogen, Hydroxy, Alkyl, Alkoxy, stehen,
oder die Verbindung PF 1022 der folgenden Formel (IIa) 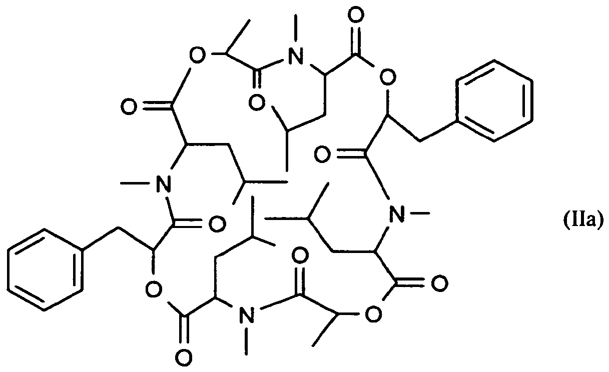 oder eine Verbindung der Formel (IIb): 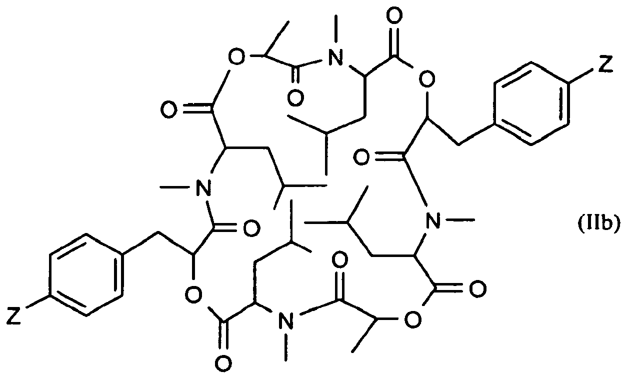 in welcher
Z für N-Morpholinyl, Amino, Mono- oder Dimethylamino steht oder
eine Verbindung der folgenden Formel (IIc): 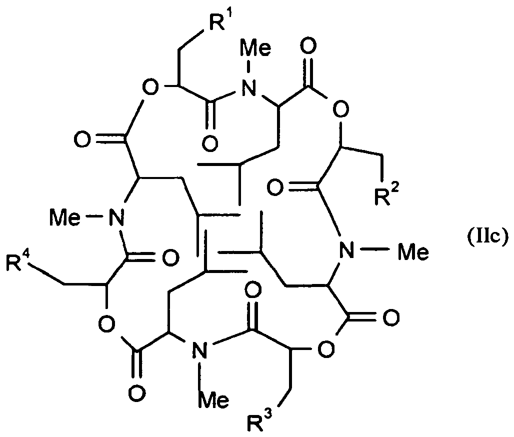 in welcher
R¹, R², R³, R⁴ unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen
oder eine Verbindung der allgemeinen Formel (IId) 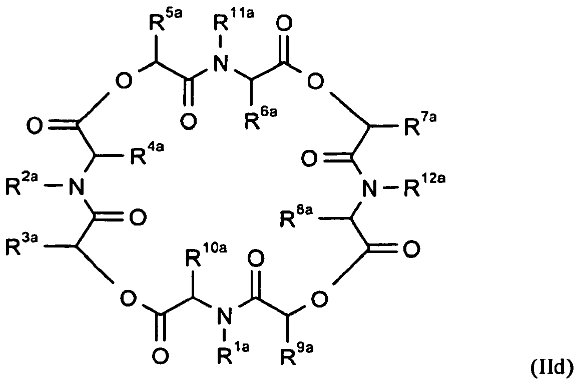 in welcher
R^{1a}, R^{2a}, R^{11a} und R^{12a} unabhängig voneinander für C₁₋₈-Alkyl, C₁₋₈-Halogenalkyl, C₃₋₆-Cycloalkyl, Aralkyl, Aryl stehen,
R^{3a}, R^{5a}, R^{7a}, R^{9a} unabhängig voneinander für Wasserstoff oder geradkettiges oder verzweigtes C₁₋₈-Alkyl steht, das gegebenenfalls durch Hydroxy, C₁₋₄-Alkoxy, Carboxy, 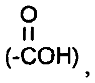 Carboxamid, 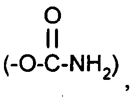 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
R^{4a} , R^{6a} , R^{8a}, R^{10a} unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen sowie deren optische Isomere und Racemate.

5. Feste Arzneimittelzubereitung gemäß Anspruch 4, enthaltend Emodepsid

6. Feste Arzneimittelzubereitung gemäß Anspruch 5, enthaltend Emodepsid und Praziquantel.

7. Feste Arzneimittelzubereitung gemäß einem der Ansprüche 1 bis 3, enthaltend ein Analgetikum.

8. Feste Arzneimittelzubereitung gemäß Anspruch 7, enthaltend Metamizol.

9. Feste Arzneimittelzubereitung gemäß einem der Ansprüche 1 bis 6, enthaltend ein Makrocyclisches Lacton.

10. Feste Arzneimittelzubereitung gemäß Anspruch 9, enthaltend Ivermectin.

11. Feste Arzneimittelzubereitung gemäß einem der Ansprüche 1 bis 3, enthaltend ein pharmakologisch verträgliches Phosphonsäurederivat ausgewählt aus Toldimfos und Butaphosphan.

12. Feste Arzneimittelzubereitung gemäß Anspruch 11, enthaltend Butaphosphan.

13. Feste Arzneimittelzubereitung gemäß einem der Ansprüche 4, 5 oder 6, **dadurch gekennzeichnet, dass** sie 80% des Depsipeptids im Paddle-Test gemäß US Pharmacopeia bei 37°C und 75 Umderhungen pro Minute als Freisetzungsmedium unter Sink-Bedingungen innerhalb von 1 bis 6 Stunden freisetzt, wobei als Freisetzungsmedium Dinatriumhydrogenphosphat-Dihydrat/Citronensäure-Monohydrat-Puffer pH 3.0 mit 0,5 % Natriumlaurylsulfat eingesetzt wird.

14. Feste Arzneimittelzubereitung gemäß Anspruch 8 **dadurch gekennzeichnet, dass** sie 80% des Metamizols im Paddle-Test gemäß US Pharmacopeia bei 37°C und 75 Umdrehungen pro Minute unter Sink-Bedingungen innerhalb von 1 bis 6 Stunden freisetzt, wobei als Freisetzungsmedium Phosphatpuffer pH 6,8 (Standardfreisetzungsmedium nach USP) eingesetzt wird.

## Claims

1. Solid pharmaceutical preparation with delayed release, comprising:
a. at least one pharmaceutically active ingredient,
b. from 15 to 40% by weight of polyvinylpyrrolidone and/or polyvinylpyrrolidone derivative, being a copolymer of vinylpyrrolidone and vinyl acetate in the ratio 6:4, the polyvinylpyrrolidone and/or polyvinylpyrrolidone derivative being a mixture
i. of a short-chain polyvinylpyrrolidone or polyvinylpyrrolidone derivative having a K value of from 17 to 30 and
ii. of a longer-chain polyvinylpyrrolidone or polyvinylpyrrolidone derivative having a K value above 40,
c. at least one filler.

2. Solid pharmaceutical preparation according to Claim 1, additionally comprising a disintegrant.

3. Solid pharmaceutical preparation according to Claim 2, comprising the disintegrant in amounts of up to 5% (m/m).

4. Solid pharmaceutical preparation according to any of the preceding claims, comprising as depsipeptide a compound of the general formula (I) : 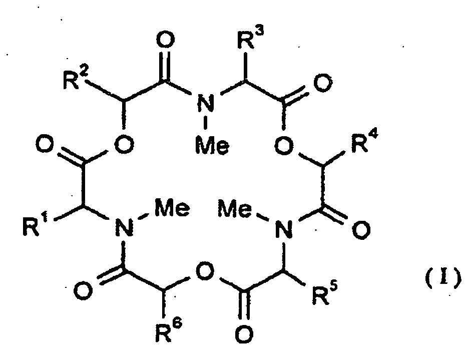 in which
R¹, R³ and R⁵ are independently of one another hydrogen, straight-chain or branched alkyl having up to 8 carbon atoms, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which may optionally be substituted by one or two benzyloxycarbonyl radicals or by one, two, three or four alkyl radicals, alkoxylcarbonylaminoalkyl, 9-fluorenylmethoxycarbonyl(Fmoc)aminoalkyl, alkenyl, cycloalkyl, cycloalkylalkyl and optionally substituted arylalkyl, where halogen, hydroxyl, alkyl and alkoxy may be mentioned as substitutents,
R², R⁴ and R⁶ are independently of one another hydrogen, straight-chain or branched alkyl having up to 8 carbon atoms, hydroxyalkyl, mercaptoalkyl, alkanoyloxyalkyl, alkoxyalkyl, aryloxyalkyl, alkylthioalkyl, alkylsulphinylalkyl, alkylsulphonylalkyl, carboxyalkyl,
alkoxycarbonylalkyl, arylalkoxycarbonylalkyl, carbamoylalkyl, aminoalkyl, alkylaminoalkyl, dialkylaminoalkyl, alkoxycarbonylaminoalkyl, alkenyl, cycloalkyl, cycloalkylalkyl, optionally substituted aryl or arylalkyl, where halogen, hydroxyl, alkyl, alkoxy may be mentioned as substituents,
or the compound PF 1022 of the following formula (IIa) 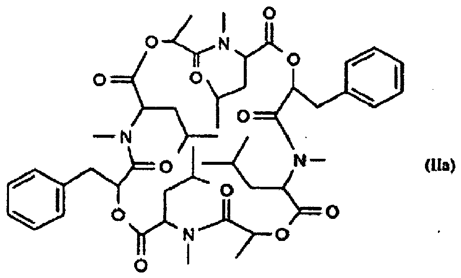 or a compound of the formula (IIb): 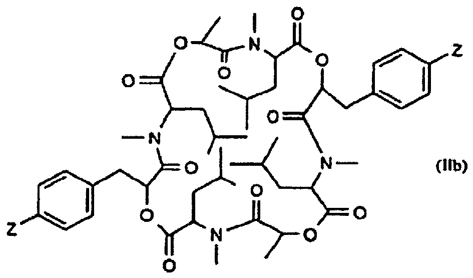 in which
Z is N-morpholinyl, amino, mono- or dimethylamino or
a compound of the following formula (IIc): 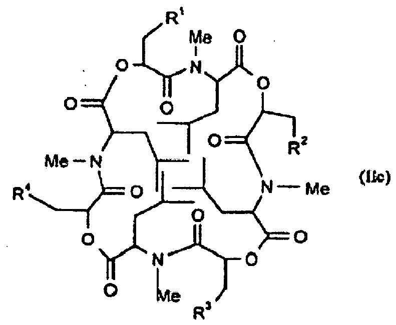 in which
R¹, R², R³, R⁴ are independently of one another hydrogen, C₁-C₁₀-alkyl or aryl, in particular phenyl, which are optionally substituted by hydroxyl, C₁-C₁₀-alkoxy or halogen
or a compound of a general formula (IId) 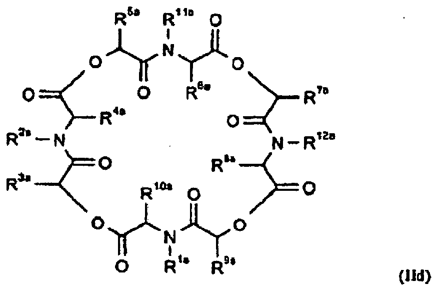 in which
R^{1a}, R^{2a}, R^{11a} and R^{12a} are independently of one another C₁₋₈-alkyl, C₁₋₈-haloalkyl, C₃₋₆-cycloalkyl, aralkyl, aryl,
R^{3a}, R^{5a}, R^{7a}, R^{9a} are independently of one another hydrogen or straight-chain or branched C₁₋₈-alkyl which may optionally be substituted by hydroxyl,
C₁₋₄-alkoxy, carboxyl 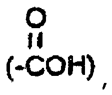 carboxamide 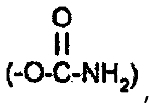 imidazolyl, indolyl, guanidino, -SH or C₁₋₄-alkylthio, and is furthermore aryl or aralkyl which may be substituted by halogen, hydroxyl, C₁₋₄-alkyl, C₁₋₄-alkoxy,
R^{4a}, R^{6a}, R^{8a}, R^{10a} are independently of one another hydrogen, straight-chain C₁₋₅-alkyl, C₂₋₆-alkenyl, C₃₋₇-cycloalkyl, each of which may optionally be substituted by hydroxyl, C₁₋₄-alkoxy, carboxyl, carboxamide, imidazolyl, indolyl, guanidino, SH or C₁₋₄-alkylthio, and are aryl or aralkyl which may be substituted by halogen, hydroxy, C₁₋₄-alkyl, C₁₋₄-alkoxy, and the optical isomers and racemates thereof.

5. Solid pharmaceutical preparation according to Claim 4, comprising emodepside.

6. Solid pharmaceutical preparation according to Claim 5, comprising emodepside and praziquantel.

7. Solid pharmaceutical preparation according to any of Claims 1 to 3, comprising an analgesic.

8. Solid pharmaceutical preparation according to Claim 7, comprising metamizole.

9. Solid pharmaceutical preparation according to any of Claims 1 to 6, comprising a macrocyclic lactone.

10. Solid pharmaceutical preparation according to Claim 9, comprising ivermectin.

11. Solid pharmaceutical preparation according to any of Claims 1 to 3, comprising a pharmacologically acceptable phosphonic acid derivative selected from toldimfos and butaphosphan.

12. Solid pharmaceutical preparation according to Claim 11, comprising butaphosphan.

13. Solid pharmaceutical preparation according to any of Claims 4, 5 or 6, **characterized in that** it releases 80% of the depsipeptide in the paddle test of the US Pharmacopeia at 37°C and 75 revolutions per minute under sink conditions within 1 to 6 hours, the release medium used being disodium hydrogen phosphate dihydrate/citric acid monohydrate buffer, pH 3.0, with 0.5% sodium lauryl sulphate.

14. Solid pharmaceutical preparation according to Claim 8, **characterized in that** it releases 80% of the metamizole in the paddle test of the US Pharmacopeia at 37°C and 75 revolutions per minute under sink conditions within 1 to 6 hours, the release medium used being phosphate buffer, pH 6.8 (USP standard release medium).

## Revendications

1. Composition pharmaceutique solide à libération retardée, contenant :
a. au moins une substance active à activité pharmaceutique,
b. 15 à 40 % en poids de polyvinylpyrrolidone et/ou d'un dérivé de polyvinylpyrrolidone, consistant en un copolymère de vinylpyrrolidone et acétate de vinyle en le rapport 6:4, la polyvinylpyrrolidone et/ou le dérivé de polyvinylpyrrolidone consistant en un mélange
i. d'une polyvinylpyrrolidone ou d'un dérivé de polyvinylpyrrolidone, à courte chaîne, ayant un indice K de 17 à 30 et
ii. d'une polyvinylpyrrolidone ou d'un dérivé de polyvinylpyrrolidone, à plus longue chaîne, ayant un indice K de plus de 40,
c. au moins un excipient.

2. Composition pharmaceutique solide selon la revendication 1, en outre contenant un désintégrant.

3. Composition pharmaceutique solide selon la revendication 2, contenant le désintégrant en quantités de jusqu'à 5 % (m/m).

4. Composition pharmaceutique solide selon l'une quelconque des revendications précédentes, contenant en tant que depsipeptide un composé de formule générale (I) : 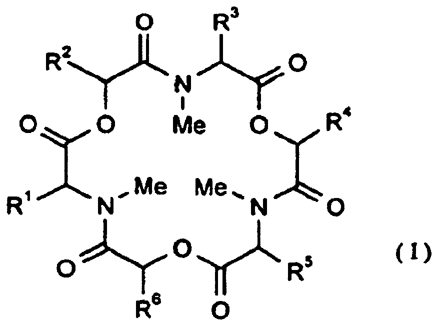 dans laquelle
R¹, R³ et R⁵ représentent, chacun indépendamment, un atome d'hydrogène, un groupe alkyle à chaîne droite ou ramifiée ayant jusqu'à 8 atomes de carbone, hydroxyalkyle, alcanoyloxyalkyle, alcoxyalkyle, aryloxyalkyle, mercaptoalkyle, alkylthioalkyle, alkylsulfinylalkyle, alkylsulfonylalkyle, carboxy-alkyle, alcoxycarbonylalkyle, arylalcoxycarbonylalkyle, carbamoylalkyle, aminoalkyle, alkylaminoalkyle, dialkylaminoalkyle, guanidinoalkyle, qui peut éventuellement être substitué par un ou deux radicaux benzyloxycarbonyle ou par un, deux, trois ou quatre radicaux alkyle, un groupe alcoxycarbonylaminoalkyle, 9-fluorénylméthoxycarbonyl(Fmoc)aminoalkyle, alcényle, cycloalkyle, cycloalkylalkyle ainsi qu'arylalkyle éventuellement substitué, comme substituants étant nommés halogène, hydroxy, alkyle et alcoxy,
R², R⁴ et R⁶ représentent, chacun indépendamment, un atome d'hydrogène, un groupe alkyle à chaîne droite ou ramifiée ayant jusqu'à 8 atomes de carbone, hydroxyalkyle, mercaptoalkyle, alcanoyloxyalkyle, alcoxyalkyle, aryloxyalkyle, alkylthioalkyle, alkylsulfinylalkyle, alkylsulfonylalkyle, carboxyalkyle, alcoxycarbonylalkyle, arylalcoxycarbonylalkyle,
carbamoylalkyle, aminoalkyle, alkylaminoalkyle, dialkylaminoalkyle, alcoxycarbonylaminoalkyle, alcényle, cycloalkyle, cycloalkylalkyle, un groupe aryle ou arylalkyle éventuellement substitué, comme substituants étant nommés halogène, hydroxy, alkyle et alcoxy,
ou le composé PF 1022 de formule (IIa) suivante 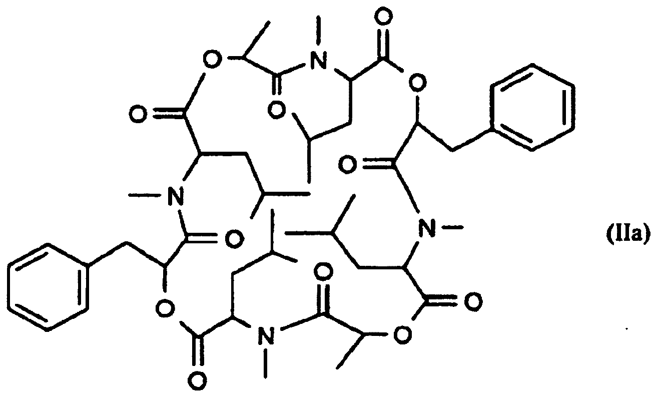 ou un composé de formule (IIb) : 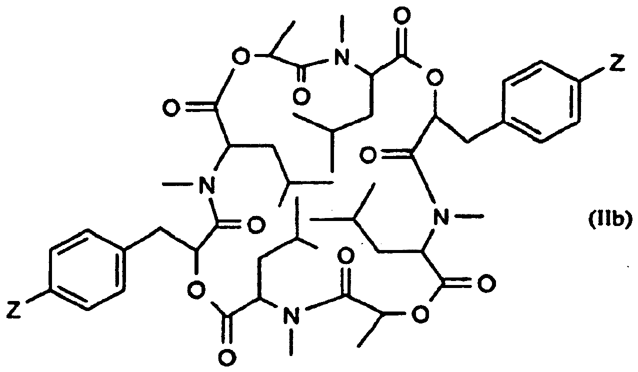 dans laquelle
Z représente un groupe N-morpholinyle, amino, mono- ou diméthylamino
ou
un composé de formule (IIc) suivante : 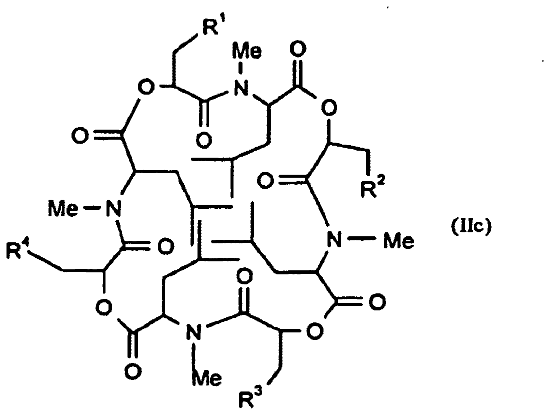 dans laquelle
R¹, R², R³, R⁴ représentent indépendamment les uns des autres un atome d'hydrogène, un groupe alkyle en C₁-C₁₀ ou aryle, en particulier phényle, qui sont éventuellement substitués par hydroxy, alcoxy en C₁-C₁₀ ou halogène
ou un composé de formule générale (IId) 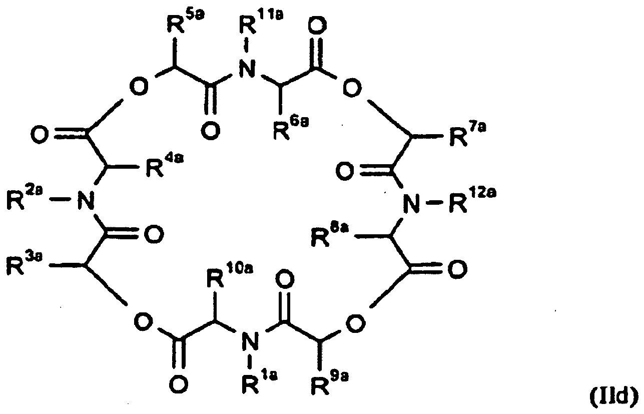 dans laquelle
R^{1a}, R^{2a}, R^{11a} et R^{12a} représentent, indépendamment les uns des autres, un groupe alkyle en C₁-C₈, halogénoalkyle en C₁-C₈, cycloalkyle en C₃-C₆, aralkyle, aryle.
R^{3a}, R^{5a}, R^{7a}, R^{9a} représentent, indépendamment les uns des autres, un atome d'hydrogène ou un groupe alkyle en C₁-C₈ à chaîne droite ou ramifiée, qui peut être éventuellement substitué par hydroxy, alcoxy en C₁-C₄,
carboxy 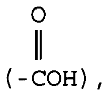 carboxamido 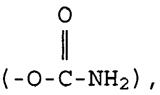 imidazolyle, indolyle, guanidino, -SH ou alkyl(C₁-C₄)thio et représentent encore un groupe aryle ou un groupe aralkyle qui peuvent être substitués par halogène, hydroxy, alkyle en C₁-C₄, alcoxy en C₁-C₄,
R^{4a} , R^{6a}, R^{8a} , R^{10a} représentent, indépendamment les uns des autres, un atome d'hydrogène, un groupe alkyle en C₁-C₅ à chaîne droite, alcényle en C₂-C₆, cycloalkyle en C₃-C₇, qui peuvent éventuellement être substitués par hydroxy, alcoxy en C₁-C₄, carboxy, carboxamido, imidazolyle, indolyle, guanidino, SH ou alkyl(C₁-C₄)thio, ainsi qu'un groupe aryle ou un groupe aralkyle qui peuvent être substitués par halogène, hydroxy, alkyle en C₁-C₄, alcoxy en C₁-C₄, ainsi que leurs isomères optiques et racémates.

5. Composition pharmaceutique solide selon la revendication 4, contenant de l'émodepside.

6. Composition pharmaceutique solide selon la revendication 5, contenant de l'émodepside et du praziquantel.

7. Composition pharmaceutique solide selon l'une quelconque des revendications 1 à 3, contenant un analgésique.

8. Composition pharmaceutique solide selon la revendication 7, contenant du métamizole.

9. Composition pharmaceutique solide selon l'une quelconque des revendications 1 à 6, contenant une lactone macrocyclique.

10. Composition pharmaceutique solide selon la revendication 9, contenant de l'ivermectine.

11. Composition pharmaceutique solide selon l'une quelconque des revendications 1 à 3, contenant un dérivé d'acide phosphonique pharmacologiquement acceptable choisi parmi le toldimphos et le butaphospane.

12. Composition pharmaceutique solide selon la revendication 11, contenant du butaphosphane.

13. Composition pharmaceutique solide selon l'une quelconque des revendications 4, 5 ou 6, **caractérisée en ce que** dans le Paddle-Test selon la Pharmacopée US à 37°C et 75 tours par minute elle libère en l'espace de 1 à 6 heures 80 % du depsipeptide dans des conditions immergées, du tampon monohydrogénophosphate de sodium dihydraté/acide citrique monohydraté pH 3,0 avec 0,5 % de laurylsulfate de sodium étant utilisé comme milieu de libération.

14. Composition pharmaceutique solide selon la revendication 8, **caractérisée en ce que** dans le Paddle-Test selon la Pharmacopée US à 37°C et 75 tours par minute elle libère en l'espace de 1 à 6 heures 80 % du métamizole dans des conditions immergées, du tampon phosphate pH 6,8 (milieu de libération standard selon l'USP) étant utilisé come milieu de libération.
